# EUROPÄISCHE PATENTSCHRIFT

(11) **EP 1 497 308 B1**
(45) Veröffentlichungstag und Bekanntmachung des Hinweises auf die Patenterteilung: **20.09.2006**
(21) Anmeldenummer: 03747107.5
(22) Anmeldetag: 22.04.2003
(51) Int. Cl.: C07J 1/00

(54) **VERFAHREN ZUM HERSTELLEN VON KRISTALLEN VON STEROIDEN, DANACH ERHÄLTLICHE KRISTALLE UND DEREN VERWENDUNG IN PHARMAZEUTISCHEN FORMULIERUNGEN**
METHOD FOR PRODUCING STEROID CRYSTALS, CRYSTALS OBTAINED BY SAID METHOD AND THEIR USE IN PHARMACEUTICAL FORMULATIONS
PROCEDE DE PRODUCTION DE CRISTAUX DE STEROIDES, CRISTAUX OBTENUS SELON CE PROCEDE ET UTILISATION DE CES CRISTAUX DANS DES FORMULATIONS PHARMACEUTIQUES

(30) Priorität: 23.04.2002 DE 10218107
(43) Veröffentlichungstag der Anmeldung: 19.01.2005
(73) Patentinhaber: Schering Aktiengesellschaft, 13353 Berlin (DE)
(72) Erfinder: GRAWE, Detlef, 99510 Kleinromstedt (DE); GERECKE, Hagen, 07743 Jena (DE); HÖSEL, Peter, 07745 Jena (DE); EICHARDT, Annette, 07616 Bürgel (DE); GLIESING, Sabine, 07743 Jena (DE); MÜLLER, Uwe, 07747 Jena (DE)
(74) Vertreter: Geyer, Fehners & Partner
(86) Internationale Anmeldenummer: PCT/EP2003/004154
(87) Internationale Veröffentlichungsnummer: WO 2003/091272

(56) Entgegenhaltungen:
- EP-A- 0 400 522
- WO-A-90/03782
- WO-A-92/08730
- STECKEL H ET AL: "MICRONIZING OF STEROIDS FOR PULMONARY DELIVERY BY SUPERCRITICAL CARBON DIOXIDE" INTERNATIONAL JOURNAL OF PHARMACEUTICS, AMSTERDAM, NL, Bd. 152, Nr. 1, 1997, Seiten 99-110, XP002052065 ISSN: 0378-5173 in der Anmeldung erwähnt

## Beschreibung

Die Erfindung bezieht sich auf ein Verfahren zum Herstellen von Kristallen von Steroiden, deren durchschnittliche Partikelgröße in einem vorgegebenen Bereich liegen und deren maximale Partikelgröße einen vorgegebenen Wert nicht überschreiten, nach diesem Verfahren erhältliche Steroidkristalle und deren Verwendung in pharmazeutischen Formulierungen, insbesondere low dose Formulierungen.

Die meisten Steroide werden aus einem geeigneten Lösungsmittel kristallisiert. Bei einer konventionellen Kühlungs- oder Verdrängungskristallisation entsteht ein grobkörniges Kristallisat. Um die insbesondere bei Low Dose Formulierungen erforderliche Homogenität der Wirkstoffverteilung (CUT) und Dissolutionkinetik zu erreichen, wird dieses Kristallisat nach traditioneller Technologie in einer Strahlmühle mikronisiert. Dabei werden durchschnittliche Korngrößen von 1.5 bis 5 µm erreicht. Es erfolgt eine enorme Vergrößerung, aber auch eine thermodynamische Aktivierung der Oberfläche durch partielle Amorphisierung bzw. durch erhebliche Störungen in der Gitterstruktur. Mit dieser Verfahrensweise sind eine Reihe von Nachteilen verbunden, die in der Literatur *(Thibert and Tawashhi: 'Micronization of pharmaceutical Solids', MML-Series, Volume 1, Ch11, 328-347)* beschrieben sind und auch bei steroidalen Wirkstoffen unterschiedlich stark ausgeprägt sein können: Durch die partielle Amorphisierung wird der Wirkstoff chemisch destabilisiert. In Wechselwirkung mit den Hilfsstoffen im Arzneimittel wird die chemische Dekomposition verstärkt. Es liegt eine instabile physikalische Struktur durch Rekristallisation des amorphen Anteiles vor. Dies führt zu Verschlechterungen der Dissolutioneigenschaften und Veränderungen in der Partikelgröße während der Lagerung des Wirkstoffes, aber auch in der Arzneifertigware. Während der Mikronisierung kommt es zu Agglomeratbildungen und Verkrustungen, die zu einem unerwünschten Grobkornanteil im Mikronisat führen. Durch Mikronisierung läßt sich die Korngröße nur in sehr engen Grenzen gezielt beeinflussen. Eine Absenkung des Mahldruckes führt zwar zu einer geringfügigen Erhöhung der durchschnittlichen Partikelgröße, aber auch zu einem unerwünschten Anstieg ihrer Streubreite. Für die Funktion der Mühle ist jedoch ein gewisser Mindestdruck unbedingt erforderlich.

Für eine gezielte Herstellung physikalisch und chemisch stabiler steroidaler Wirkstoffe mit einer an die jeweilige Dosierung angepassten Partikelgrößenverteilung ist die Mikronisierung als Verfahren nur bedingt geeignet. Das gleiche gilt für alternative Verfahren wie etwa die Herstellung mikrofeiner Wirkstoffe aus überkritischen Gasen *(Steckel at al.: Micronizing of steroids for pulmonary delivery by supercritical corbon dioxide, Int. Journal of Pharmaceutics 152 (1997)99-110).* Diese Verfahren sind technologisch sehr anspruchsvoll und apparativ wegen der hohen Drücke sehr aufwendig. Die Sprühtrocknung *(Wendel at al.: ,An Overview of Spray-Drying Applications, Pharmaceutical Technology' , Oct 1997, 124-156)* ist ebenfalls geeignet, mikrofeine Partikel zu erzeugen, aber auch hier besteht die Gefahr der Erzeugung instabiler amorpher oder teilkristalliner Strukturen.

Aus der Literatur ist bekannt, dass feine Körnungen durch Fällungen aus stark übersättigten Lösungen oder mit hoher Rührerdrehzahl erzeugt werden können. *(B.Yu. Shekunov at al.: 'Crystallization process in pharmaceutical technology and drug delivery design, Journal of Crystal Growth 211 (2000) 122-136*; *Halasz-Peterfi at al.: Formation of Micropartikles of pharmaceuticals by homogeneus nucleation, Industrial Crystallization,* 1999, *1-11; Affonso at al.: Microcrystallization Methods for Aspirin, Journal of Pharmaceutical Sciences, oct. 1971, 1572-1574).*

In *US-A-3,226,389* wird eine entsprechende Methode zur Erzeugung von Mikrokristallen durch rasche Kühlung und intensive Durchmischung beschrieben. Diese Kristallisate weisen jedoch oftmals eine große Streubreite auf und enthalten grobkörnige Agglomerate. Auch ist durch das komplexe Wechselspiel von Übersättigung, primärer und sekundärer Keimbildung und Kristallwachstum bzw. Agglomeratbildung die gezielte Erzeugung einer bestimmten Partikelgrößenverteilung nur schwer möglich.

Eine weitere Möglichkeit definierte Komspektren mikrofeiner Steroidkristalle unabhängig von einer mechanischen Prozedur zu erzeugen wird in *WO-A-92*/*08730* beschrieben. Dort wird aus einem ternären Gemisch, bestehend aus einem hydrophilen und lipophilen Lösungsmittels sowie einem Surfactanten durch Kühlung ein Kristallisat erzeugt. Diese ist zwar feiner als das Ausgangsmaterial jedoch für viele Anforderungen der Low Dose Formulierungen noch zu grob und es bestehen die gleichen o.g. Nachteile, die ein Kristallisieren aus stark übersättigten Lösungen naturgemäß mit sich bringt. Hinzu kommt, dass eine Verunreinigung des Wirkstoffs mit Surfactanten in Kauf genommen werden muß.

In *EP 0 522 700* wird die zum Stand des Wissens der Kristallisationstechnik gehörende Möglichkeit beschrieben, auf vorhandene Startkristalle durch weitere definierte Abkühlung und Erwärmung eines Teilstromes, der in den Kristallisationsprozess rückgeführt wird, ein Kristallwachstum zu erreichen. Hierbei wird jedoch in erster Linie eine Kornvergröberung auf Korngrößen weit oberhalb 100 µm erzielt, um Filtrations- und Waschprozesse zur Erzielung höherer Reinheiten zu verbessern.

Der Einflusse der Partikelgröße und Form auf den CUT-Wert in festen Arzneiformen wurden für sphärische Partikel in *M.C.R. Johnson 'Parlicle size distribution of the activ ingredient for solid dosage forms of low dosage' Pharmaceutica Acta Helvetiae, 546-559, Vol.47,* 1972 und unter Berücksichtigung anderer Formen in *P.Guitard at al. ,Maximale zulässige Partikelgrößenverteilung von Wirkstoffen für feste Arzneiformen in niedriger Dosierung*'; *Pharm.Ind.36, Nr.4(1974)* beschrieben. Aus den dort aufgeführten Beziehungen können die maximalen Partikelabmessungen bezogen auf die jeweilige Dosis berechnet werden.

Die Dissolutionkinetik ist ein weiterer wichtiger Parameter zur Bewertung der meist schlecht wasserlöslichen steroidalen Mikrokristalle.

Die pharmazeutische Eignung muß stets durch entsprechende standardisierte Tests nachgewiesen werden. Das gleiche gilt für die Stabilität der Mikrokristalle als Wirkstoff und im Arzneimittel.

Eine Kritik aller aufgeführten Verfahren zur Erzeugung von Mikrokristallen in Suspensionen für Low Dose Formulierungen, besteht in der Isolierung und Trocknung. Es ist sehr problematisch derartige feinkörnige, feuchte Kristalliate zu trocknen, ohne die Korngrößenverteilung zu beeinträchtigen.

Der vorliegenden Erfindung liegt somit die Aufgabe zugrunde, ein Verfahren zum Herstellen von Steroidkristallen bereitzustellen, das nicht die aus dem Stand der Technik bekannten Nachteile aufweist und mit dem insbesondere Kristalle erhältlich sind, die die Anforderungen an low dose Formulierungen erfüllen.

Erfindungsgemäß wird dies erreicht durch ein Verfahren zum Herstellen von Kristallen von Steroiden, deren durchschnittliche Partikelgröße 1 µm bis 25 µm betragen und deren maximale Partikelgröße 100 µm nicht überschreiten, wobei eine übersättigte Lösung des Steroids während der Kristallisation einem Naßmahlen mittels einer Vorrichtung zum Naßmahlen unterzogen wird, wodurch eine Primärkomsuspension erhalten wird.

Unter dem Ausdruck "Steroid" werden natürlich vorkommende und synthetische Verbindungen verstanden, denen als Gerüst des (partiell) hydrierten Cyclopenta[α]phenanthrens zugrunde liegt. Beispiel eines Steroids ist 11β-{4-[(Ethylaminocarbonyl)oximinomethyl]phenyl}-17β-methoxy-17α-methoxymethyl-estra-4,9-dien-3-on (nachfolgend als J956 bezeichnet), anhand dem beispielhaft die vorliegende Erfindung erläutert wird.

Mit dem erfindungsgemäßen Verfahren ist es in überraschender Weise möglich, Kristalle zu erhalten, die ausreichend stabil sind und hinsichtlich der Parameter ihrer Partikelgrößenverteilung den pharmazeutischen Anforderungen bezüglich Homogenität der Wirkstoffverteilung (CUT) und Dissolutionskinetik für low dose Formulierungen eingestellt und somit gerecht werden können. Des weiteren kann eine für die jeweilige Dosis geeignete Korngrößenverteilung mit hoher Zielgenauigkeit und Reproduzierbarkeit hergestellt werden. Ferner kann das erfindungsgemäße Verfahren in einfacher, schneller und kostengünstiger Weise durchgeführt werden. Die Steroidkristalle können vorteilhafterweise ohne Beeinträchtigung ihrer Korngrößenverteilung aus Suspension isoliert und getrocknet werden.

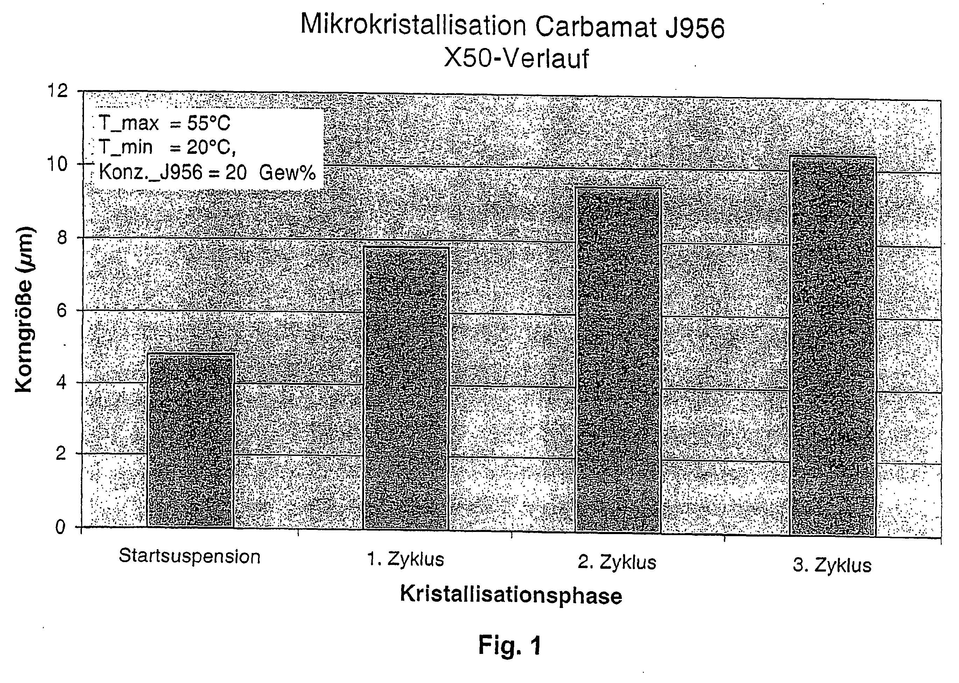

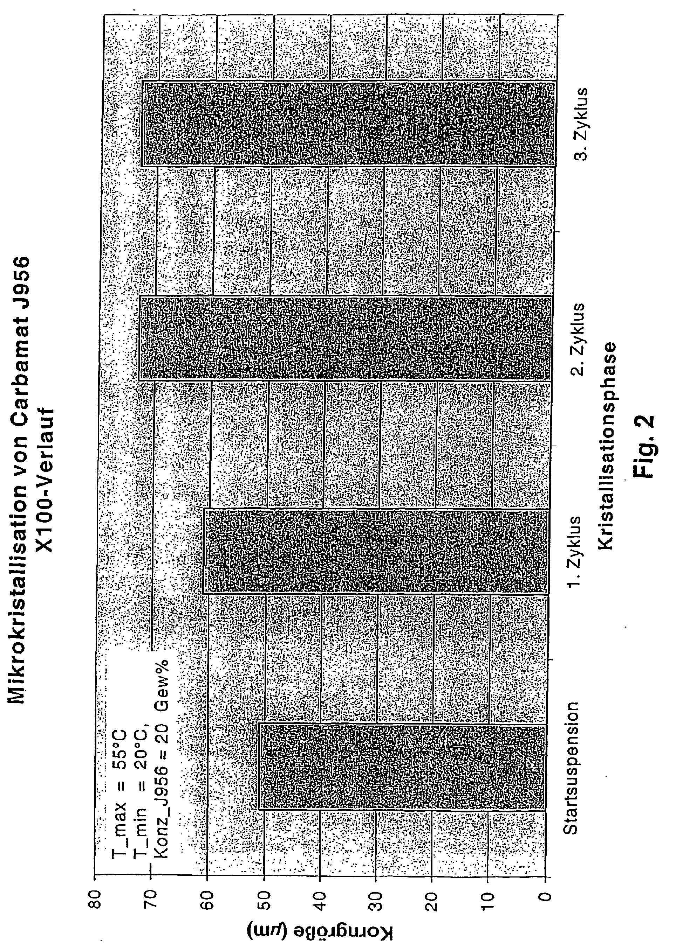

Die Erfindung wird nachstehend unter Bezugnahme auf die Figuren näher erläutert, wobei
- Fig. 1 und 2: die Entwicklung der Korngröße beim erfindungsgemäßen Kristallisationsverfahren zeigen.

Vorzugsweise beträgt die durchschnittliche Partikelgröße 7 µm bis 15 µm. Die maximale Partikelgröße überschreitet vorzugsweise nicht 80 µm. Der Ausdruck maximale Partikelgröße" bedeutet dabei, daß kein Teilchen größer als der angegebene Wert ist. Innerhalb dieser Grenzen der durchschnittlichen Partikelgröße und der maximalen Partikelgröße ist es in günstiger Weise möglich, die Partikelgrößenverteilung so zu wählen, daß sie den pharmazeutischen Anforderungen bezüglich CUT und Dissolutionskinetik für low dose Formulierungen entspricht.

Im erfindungsgemäßen Verfahren wird eine übersättigte Lösung eines Steroids eingesetzt. Die Lösung enthält als Gelöstes das Steroid, das in einem Lösungsmittel dafür gelöst ist. Als Lösungsmittel werden auch Gemische verschiedener Lösungsmittel verstanden. Eine im erfindungsgemäßen Verfahren eingesetzte übersättigte Lösung, die beispielsweise durch Unterkühlung hergestellt werden kann, enthält mehr gelösten Stoff als sie in ihrem thermischen Gleichgewicht aufweisen dürfte. Es können im erfindungsgemäßen Verfahren übersättigte Lösungen eingesetzt werden, in denen Keime spontan gebildet werden.

In einer bevorzugten Ausführungsform des erfindungsgemäßen Verfahrens enthält die übersättigte Lösung 1 Gew.-% bis 50 Gew.-%, vorzugsweise 5 Gew.-% bis 35 Gew.-%, des Steroids, bezogen auf die übersättigte Lösung. Mit diesen übersättigten Lösungen können die vorstehend beschriebenen Vorteile des erfindungsgemäßen Verfahrens in besonders günstiger Weise erreicht werden.

Die Herstellung der übersättigten Lösungen kann in üblicher Weise erfolgen. Vorzugsweise wird die übersättigte Lösung hergestellt durch Auflösen des Steroids in einem Lösungsmittel bei einer Temperatur unterhalb des Siedepunkts und nachfolgendem Abkühlen auf eine Temperatur oberhalb des Gefrierpunkts der Lösung. Wird für das erfindungsgemäße Verfahren das Steroid J956 Ethylacetat als Lösungsmittel für die übersättigte Lösung eingesetzt, kann das Erwärme auf beispielsweise etwa 70°C erfolgen, bis das Steroid im Ethylacetat klar gelöst ist. Das Abkühlen kann während 10 Minuten bis 1 Stunde, insbesondere 15 Minuten bis 30 Minuten, auf etwa 35°C erfolgen. Der Fachmann kann durch einfache Tests aufgrund vorstehender Angaben die Parameter zur Herstellung einer übersättigten Lösung mit einem anderen Lösungsmittel als Ethylacetat und einem anderen Steroid als J956 ohne weiteres ermitteln.

Günstigerweise wird die Kristallisation in einem Gefäß durchgeführt, das einen Rührer aufweist. Beispiel dafür sind die für technische Anwendungen an sich bekannten Kristallisatoren.

Im erfindungsgemäßen Verfahren erfolgt während der Kristallisation ein Naßmahlen mittels einer Vorrichtung zum Naßmahlen. Die Kristallisation aus der übersättigten Lösung kann einsetzen, nachdem mit dem Naßmahlen begonnen wurde. Geeignete Vorrichtungen für den Schritt des Naßmahlens sind Dispergierwerkzeuge und Homogenisatoren, wie Rotor-Stator-Werkzeuge, Rührwerksmühlen, Walzenstühle und Kolloidmühlen.

Die erfindungsgemäße Herstellung der Kristalle erfolgt, wie bereits vorstehend beschrieben, durch Kristallisation aus einem Lösungsmittel oder Lösungsmittelgemisch, in dem in der Startphase der Kristallisation, entweder kurz nachdem die Kristallisation begonnen hat oder bevor sie begonnen hat, zusätzlich zum konventionellen Rührwerk ein Naßmahlen mittels einer Vorrichtung zum Naßmahlen, insbesondere eines Rotor-Stator-Werkzeugs oder einer Kolloidmühle, durchgeführt wird. Diese Vorrichtung zum Naßmahlen kann direkt als zusätzliches Rührwerk im Kristallisationsgefäß oder in einer Umlaufschleife des Kristallisators eingesetzt werden. Der Einsatz in einer Umlaufschleife ist günstig, da die Vorrichtung zum Naßmahlen gleichzeitig als Förderaggregat wirkt. Wird ein Rotor-Stator-Werkzeug verwendet, so kann die Rotorumfangsgeschwindigkeit 10 m/s bis 50 m/s, vorzugsweise 20 m/s bis 40 m/s betragen. Durch den durch das Naßmahlen, insbesondere den Rotor-Stator, bewirkten zusätzlichen Energieeintrag wird eine sehr hohe sekundäre Keimbildungsrate erzeugt und dadurch das Kristallwachstum stark eingeschränkt. Zusätzlich werden sich evtl. bildende Agglomerate im engen Scherspalt zerschlagen. Somit wird ein feines Primärkorn erzeugt, dessen durchschnittliche Partikelgröße je nach eingestellter Übersättigung und Rotorumfangsgeschwindigkeit zwischen 3 µm und 25 µm beträgt und dessen maximale Partikelgröße 25 µm bis 80 µm nicht übersteigt. Diese Partikelparameter können für low dose Formulierungen bereits ausreichend sein.

Um entsprechend den pharmazeutischen Anforderungen auch gröbere Kömungen mit definierter Partikelgrößenverteilung mit entsprechender Zielgenauigkeit und hoher Reproduzierbarkeit herstellen zu können, wird die Primärkornsuspension vorzugsweise einem oszillatorischen Temperaturprofil unterworfen. Hierzu wird die erzeugte feine Primärkornsuspension auf eine Temperatur Tₘₐₓ unterhalb der Löslichkeitsgrenze der Primärkörner in der Suspension erwärmt und nachfolgend langsam auf eine Temperatur Tₘᵢₙ, die oberhalb des Gefrierpunkts der Suspension liegt, abgekühlt. Beim Aufwärmvorgang wird die Feinkornfraktion der Primärkornsuspension aufgelöst und bei einem anschließenden Kühlvorgang auf die vorhandene Grobkomfraktion aufkristallisiert. Hierdurch ergibt sich eine definierte Verschiebung der Partikelgrößenverteilung zum gröberen Bereich. Vorzugsweise wird Tₘₐₓ so gewählt, daß 10 Gew.% bis 95 Gew.%, insbesondere 20 Gew.% bis 50 Gew.-%, ganz besonders etwa 30 Gew.-% der Primärkömer im Lösungsmittel aufgelöst werden. Der Anteil der aufzulösenden Menge der Primärkömer wird in Abhängigkeit von der vorgegebenen Körnung gewählt, die wiederum durch die Art der low dose Formulierung bestimmt ist. Wird ein hoher Anteil der Primärkörner aufgelöst, wird eine gröbere Kömung erhalten.

In einer bevorzugten Ausführungsform des erfindungsgemäßen Verfahrens wird Tₘᵢₙ so gewählt, daß die aufgelösten Primärkömer im wesentlichen wieder kristallisieren. Günstigerweise sollten, um die Verluste an Steroiden gering zu halten, nahezu alle der aufgelösten Primärkörner an den noch verbleibenden Primärkömem kristallisieren.

Vorzugsweise erfolgt das Abkühlen von Tₘₐₓ auf Tₘᵢₙ während 1 Minute bis 10 Stunden, insbesondere während 0,5 Stunden bis 2 Stunden.

Die Abkühlflanke des Temperaturprofils sollte dabei so gesteuert werden, daß die erneute Keimbildung möglichst gering gehalten wird. Die Schrittweite dieser Vergröberung ist abhängig von dem im Heizzyklus aufgelösten Mengenanteil des Kristallisates, welcher wiederum von Lage der beiden Temperaturen Tₘₐₓ und Tₘᵢₙ in bezug auf die Löslichkeitsgrenze und von der Feststoffkonzentration der Suspension bestimmt ist. Dieser Heiz-Kühlzyklus kann so oft wiederholt werden, vorzugsweise 1 bis 20 mal, bis die gewünschte Partikelgrößenverteilung erreicht wird. Steuerparameter sind dabei Tₘₐₓ, Tₘᵢₙ und die Anzahl der Zyklen. Je geringer die gewünschte Vergröberung, um so geringer sollte Tₘₐₓ gewählt werden. Somit kann man sich in kleinen Schritten der gewünschten Endkörnung annähern. Der Verlauf des aufgelösten Anteils des Kristallisates in den Heizperioden wird dabei so dimensioniert, daß der maximale Partikeldurchmesser nur noch in sehr geringem Maße zunimmt und die Vergröberung im Bereich der feineren Partikeln stattfindet. So wird beispielsweise bei Auflösung und Rekristallisieren von 40% der aus einer 20 Gew%-igen Essigesterlösung auskristallisierten J956-Menge der durchschnittliche Partikeldurchmesser (X50) von 4.9 µm auf 7.8 µm erhöht, während die Vergrößerung der maximalen Korngröße (X100) kaum meßbar ist. Dies bedeutet, daß die Partikelgrößenverteilung bei Wachstum ihres Durchschnittwertes (X50) deutlich enger wird. Bezüglich der pharmazeutischen Verwendung, insbesondere für die Erzielung entsprechender CUT-Werte und Dissolutionseigenschaften, ist dieser Effekt besonders vorteilhaft.

Erfindungsgemäß kann durch entsprechende Wahl des Werkzeuges und der Prozessbedingungen durch diese Kombination zweier Prozesse ein sehr feines und enges Komspektrum gewonnen werden, da durch den überlagerten Kristallisationsprozess der für Mahlprozesse oftmals typische hohe Feinkomanteil reduziert wird. Die maximale Korngröße kann sehr kleingehalten werden, da die Agglomeratbildung weitgehend vermieden wird.

Nach der Durchführung des oszillatorischen Temperaturprofils kann die erhaltene Kristallsuspension filtriert und mit einem Lösungsmittel gewaschen werden, indem das Steroid nur in geringen Mengen von beispielsweise weniger als 1 Gew.-% löslich ist. Beispiele solcher Lösungsmittel sind Methyl-tert.-butylether, Hexan, Heptan, Wasser oder Gemische von zwei oder mehreren dieser. Dadurch wird beim anschließenden Trocknungsprozeß, der vorteilhafterweise direkt in der Filtrationseinheit durch ein Trocknungsgas oder im Vakuum erfolgt, eine Brückenbildung und Agglomeration der Partikel vermieden.

Die Trocknung kann durch Konvektions- oder Vakuumtrocknung in ruhender oder bewegter Schüttung erfolgen.

Wenn eine konventionelle Filtration und Trocknung schwer möglich ist und zu einer Beeinträchtigung der bei der Kristallisation erzeugten Partikelgrößenverteilung führt, wie beispielsweise im Falle sehr feiner Körnungen, kann alternativ der filtrierte und gewaschene Filterkuchen mit einer Suspendierflüssigkeit mit sehr geringer Löslichkeit für das Steroid, beispielsweise weniger als 1 Gew.-%, vorzugsweise Wasser, aufgeschlämmt werden. Die erhaltene Suspension kann über Sprühtrocknung in die getrocknete feste Form des Steroids überführt werden.

Gegenstand der vorliegenden Erfindung sind weiterhin Steroidkristalle, die nach dem erfindungsgemäßen Verfahren erhältlich sind. Zur Durchführung des Verfahrens wird auf die vorstehenden Ausführungen, in denen das erfindungsgemäße Verfahren im Detail beschrieben wurde, verwiesen.

Die vorliegende Erfindung betrifft ferner pharmazeutische Formulierungen, die die nach dem erfindungsgemäßen Verfahren erhältlichen Steroidkristalle aufweisen.

Ein Beispiel einer geeigneten Kapselrezeptur ist in Tabelle 1 angegeben:

**Tab 1: Zusammensetzung einer geeigneten Kapselrezeptur der Dosierung 1 mg J956**

| Substanz | Menge |
|---|---|
| J956, mikrokristallin | 1,000 mg |
| mikrokristalline Cellulose | 102,480 mg |
| Magnesiumstearat | 0,520 mg |
| Hartgelatinekapsel, Größe 3 | 1 Stück |
| Kapselfüllmasse | 104,000 mg |

In Tabelle 2 ist ein Beispiel einer geeigneten Tablettenrezeptur angegeben:

**Tab 2: Zusammensetzung einer geeigneten Tablettenrezeptur der Dosierung 1 mg J956**

| **Kern:** | |
|---|---|
| J956, mikrokristallin | 1,00 mg |
| Laktose-Monohydrat | 33,8 mg |
| Maisstärke | 18,0 mg |
| Maltodextrin (10 % in Wasser) | 6,0 mg |
| Na-Carboxymethylstärke | 0,6 mg |
| Glycerol-Monobehenat | 0,6 mg |

| **Hülle:** | |
|---|---|
| Hydroxypropylmethylcellulose | 1.125 mg |
| Talkum | 0.225 mg |
| Titandioxid | 0.625 mg |
| Eisenoxid, Pigment gelb | 0.020 mg |
| Eisenoxid, Pigment rot | 0.005 mg |

Ein wesentliches Ergebnis der Erfindung besteht darin, daß Mikrokristalle der Steroide erhältlich sind, die chemisch deutlich stabiler sind als bisher bekannte Mikronisate, da sie zum einen eine geringere spezifische Oberfläche und zum anderen eine durch den erfindungsgemäßen Kristallisationsprozeß ungestörte und hochkristalline Oberfläche aufweisen.

Ein weiteres Ergebnis ist, daß die nach dem erfindungsgemäßen Verfahren erhältlichen Mikrokristalle der Steroide bezüglich ihrer Korngrößenverteilung und Löslichkeitseigenschaften den pharmazeutischen Anforderungen der Arzneifertigware bezüglich CUT und Dissolution entsprechen.

Am Beispiel der 1 mg Kapsel und 1 mg Tablette (vergleiche vorstehend) konnte gezeigt werden, daß die erreichten Werte denen bei Verwendung von mikronisiertem Festkörper nicht nachstehen (Tab. 3, Tab. 4).

**Tab 3: J956: Freisetzung aus 1mg Kapseln mit mikrokistallinem Festkörper im vgl. zu Kapseln mit mikronisiertem Wirkstoff**

| Testmedium: 0.3% SDS in Wasser, Paddle, 100 U/min | | | | | | |
|---|---|---|---|---|---|---|
| Korndurchmesser (µm) | | Freisetzung (%) | | | | |
| X50 | X100 | 0 min | 10 min | 20 min | 30 min | 45 min |
| 3,4 | 25 | 0 | 90,7 | 97,3 | 98,1 | 99,9 |
| 5,2 | 30 | 0 | 89,8 | 93,5 | 93,4 | 95,6 |
| 6,6 | 43 | 0 | 93,2 | 95,9 | 96,7 | 96,8 |
| 8,7 | 43 | 0 | 93,5 | 96,7 | 98,5 | 99,7 |
| 14,1 | 87 | 0 | 90,2 | 95,3 | 96,0 | 96,3 |
| Mikronisat | | 0 | 92,1 | 94,3 | 94,6 | 94,9 |

**Tab 4: J956: Schwankungsbreite der CUT-Werte 1 mg Kapsel mit mikrokristallinem Festkörper im Vergleich zu Kapseln mit mikronisiertem Wirkstoff**

| Korndurchmesser (µm) | | | |
|---|---|---|---|
| X50 | X100 | Konfidenz intervall(%) | RSD(%) |
| 3,4 | 25 | 2,23 | 3,56 |
| 5,2 | 30 | 1,20 | 2,08 |
| 6,6 | 43 | 1,08 | 1,57 |
| 8,7 | 43 | 0,93 | 1,38 |
| 14,1 | 87 | 1,77 | 2,50 |
| Mikronisat | | 1,72 | 2,56 |

**Tab 5: J956: Freisetzung aus 1mg Tablette mit mikrokistallinem Festkörper im vgl. zu Tabletten mikronisiertem Wirkstoff**

| Testmedium: 0.3% SDS in Wasser, Paddle, 100 U/min | | | | | | |
|---|---|---|---|---|---|---|
| Korndurchmesser (µm) | | Freisetzung (%) | | | | |
| X50 | X100 | 0 min | 10 min | 20 min | 30 min | 45 min |
| 10,6 | 73 | 0 | 73,7 | 90,3 | 91,85 | 96,6 |
| Mikronisat | | 0 | 92,1 | 94,3 | 94,6 | 94,9 |

**Tab 6: J956: Schwankungsbreite der CUT-Werte 1 mg Tablette mit mikrokristallinem Festkörper im Vergleich zurTablette mit mikronisiertem Wirkstoff**

| Korndurchmesser (µm) | | | |
|---|---|---|---|
| X50 | X100 | Konfidenz intervall(%) | RSD(%) |
| 10,6 | 73 | 1,16 | 1,70 |
| Mikronisat | | 1,72 | 2,56 |

Ein weiteres wichtiges Resultat ist, daß mit dem erfindungsgemäßen Verfahren zielgenau und mit hoher Reproduzierbarkeit die pharmazeutisch erforderliche Korngrößenverteilung der Steroide erzeugt werden kann. In Fig. 1 und 2 ist die Entwicklung der Korngröße im Kristallisationsverfahren dargestellt. Dabei ist von Vorteil, daß sich die Streuung der Partikelgrößenverteilung deutlich vermindert und trotz Vervielfachung der durchschnittlichen Korngröße die maximale Korngröße deutlich weniger zunimmt. Dies unterstützt die Erzielung guter CUT-Werte auch in low dose Formulierungen.

Weiter wurde erreicht, daß die in Suspension erzeugte Korngrößenverteilung auch im getrockneten Festkörper erhalten bleibt.

**Tab. 7: Korngrößenverteilung vor und nach Trocknung**

| | X10 | X50 | X90 | X100 |
|---|---|---|---|---|
| Suspension* | 2,62 | 10,4 | 24 | 73 |
| nach Trocknung auf Filter | 2,7 | 10,61 | 24 | 73 |
| | | | | |

| | X10 | X50 | X90 | X100 |
|---|---|---|---|---|
| Suspension** | 2,11 | 8,6 | 19 | 51 |
| nach Sprühtrocknung | 2,25 | 8,03 | 17 | 43 |

| | | | | |
|---|---|---|---|---|
| *) Suspension J956 in Ethylacetat mit 14 Gew.-% mikrokristalle J956 **) Suspension J956 in Wasser/Ehanol (90/10 w/w) mit 10 Gew.-% Mikrokristalle J956 | | | | |

Zur Ermittlung der experimentellen Daten wurden folgende Meßverfahren eingesetzt:

### Korngrößenverteilung:

Sympatec HELOS (H0445), Trockendispergiersystem (RODOS), Druck 2 bar.

### Content Uniformity Test

Gehaltsbestimmung entsprechend USP/Ph. Eur. an Einzelkapseln nach Ausspülen durch HPLC mit externer Kalibrierung
Säule: LiChrospher 5 µ RP-18 encapped, 150 x 3 mm
Eluent: Acetonitril / Wasser = 45 / 55
Fluß: 1 ml / min
Detektion UV (272 nm)

### Wirkstofffreisetzung

Wirkstofffreisetzung in 1000 mL Wasser mit 0,3 % Natriumdodecylsulfat, 100 U/min
Gehaltsbestimmung durch HPLC mit externer Kalibrierung
Säule: LiChrospher 5 µ RP-18 encapped, 150 x 3 mm
Eluent: Acetonitril / Wasser = 45/55
Fluß: 1 ml / min
Detektion UV (272 nm)

Die nachfolgenden Beispiele dienen zur Erläuterung der Erfindung ohne sie aber darauf zu beschränken.

### Beispiel 1

In einem Glasreaktor mit Ankerrührer und Heiz/Kühldoppelmantel werden 250 g J956 in 1100 ml Ethylacetat bei 70°C klar gelöst. Die Lösung wird innerhalb von 30 min auf 35°C gekühlt. Es wird ein Rotor-Stator Dispergierwerkzeug (Ultra Turrax, T25 basic, mit S25N-25F) eingebracht und mit einer Drehzahl von 12000-18000 U/min betrieben. Nach 2-5 min setzt Kristallisation ein. Der Ultra Turrax wird noch weitere 10 min betrieben und dann abgestellt.
Die erhaltene Startsuspension wird auf 55°C aufgeheizt und anschließend innerhalb von 1h 20 min auf 20°C gekühlt. Dieser Prozess wird noch zweimal wiederholt.
Anschließend wird über eine Fritte filtriert und mit 500 ml kaltem MTBE gewaschen.
Der Filterkuchen wird mit Luft trockengesaugt.

Es wurden Mikrokristalle mit folgender Partikelgrößenverteilung erhalten:

| | Partikelgröße(µm) |
|---|---|
| X10 | 2,62 |
| X50 | 10,4 |
| X100 | 73 |

### Beispiel 2

In einem Sulfierkolben mit Blattrührer und thermostatisiertem Hiez/Kühlbad werden 50 g J956 in 200 g Ethylacetat bei 70°C klar gelöst. Die Lösung wird innerhalb von 15 min auf 35°C gekühlt. Es wird ein Rotor-Stator Dispergierwerkzeug (Ultra Turrax, T25 basic, mit S25N-25F) eingebracht und mit einer Drehzahl von 12000-16000 U/min betrieben. Nach 2 min setzt Kristallisation ein. Der Ultra Turrax wird noch weitere 10 min betrieben und dann abgestellt.
Die erhaltene Startsuspension wird auf 50°C aufgeheizt und anschließend innerhalb von 1h auf 20°C gekühlt. Dieser Prozess wird noch zweimal wiederholt.
Anschließend wird die Suspension über eine Fritte filtriert und mit 100 ml MtBE gewaschen. Der Filterkuchen wird mit 1000 ml Wasser sehr gründlich gewaschen und anschließend in 300 g Wasser aufgeschlämmt. Die Suspension wird unter folgenden Bedingungen in einem Laborsprühtrockner mit Zweistoffdüse(2 mm) (QVF/Yamato) sprühgetrocknet

| | |
|---|---|
| Trocknungsgas_Eintrittstemperatur: | 170°C |
| Trocknungsgas_Austrittstemperatur. | 60°C |
| Durchsatz Trocknungsgas : | 0.23 m3/min |
| Sprühdüse(d= 2 mm) : | 2.5 bar |
| Feed : | 8-10 ml/min |

Im Abscheidefilter des Sprühtrockners wurden Mikrokristalle mit folgender Korngrößenverteilung erhalten

| | Partikelgröße(µm |
|---|---|
| X10 | 1,75 |
| X50 | 6,04 |
| X100 | 36 |

### Beispiel 3

In einem Glasreaktor mit Ankerrührer und Heiz/Kühldoppelmantel werden 270 g J956 in 1200 ml Ethylacetat bei 75°C klar gelöst. Die Lösung wird innerhalb von 30 min auf 38°C gekühlt. Die Lösung wird vom Kristallisator-Bodenauslauf über ein externes Rotor-Stator Dispergierwerkzeug (IKA-Labor-Pilot 2000/4 mit DR-Modul) zurück in den Kristallisator gefahren. Das Dispergierwerkzeug wird mit einer Drehzahl 9000 U/min betrieben. Nach 2-5 min setzt Kristallisation ein. Das Dispergierwerkzeug wird noch weitere 10 min betrieben und dann abgestellt.
Die erhaltene Primärkomsuspension wird zweimal auf 50°C aufgeheizt und anschließend innerhalb von 1 h 20 min auf 20°C gekühlt. Dieser Prozess wird noch zweimal wiederholt. Anschließend wird über eine Fritte filtriert und mit 500 ml kaltem MTBE gewaschen.
Der Filterkuchen wird mit Luft trockengesaugt.

Es wurden Mikrokristalle mit folgender Partikelgrößenverteilung erhalten:

| | Partikelgröße(µm) | |
|---|---|---|
| | Primärkom | Ende |
| X10 | 3 | 4 |
| X50 | 9 | 13 |
| X100 | 61 | 73 |

### Beispiel 4

In einem Glasreaktor mit Ankerrührer und Heiz/Kühldoppelmantel werden 270 g J956 in 1200 ml Ethylacetat bei 75°C klar gelöst. Die Lösung wird innerhalb von 30 min auf 26°C gekühlt. Die Lösung wird vom Kristallisator-Bodenauslauf über eine externe gekühlte Kolloidmühle (IKA-Labor-Pilot 2000/4 mit Kolloidmühlenmodul) zurück in den Kristallisator gefahren. Das Dispergierwerkzeug wird mit einer Drehzahl 8900 U/min betrieben. Nach 30 sec setzt bei 36°C Kristallisation ein. Die Kolloidmühle wird noch weitere 10 min betrieben, die Suspension beprobt und dann abgestellt.
Die erhaltene Primärkornsuspension wird anschließend auf 55°C aufgeheizt und anschließend innerhalb von 2h auf 20°C gekühlt.
Anschließend wird über eine Fritte filtriert und mit 500 ml kaltem MTBE gewaschen.
Der Filterkuchen wird mit Luft trockengesaugt.

Es wurden Mikrokristalle mit folgender Partikelgrößenverteilung erhalten:

| | Partikelgröße(µm) | |
|---|---|---|
| | Primärkorn | Ende |
| X10 | 1.2 | 1.4 |
| X50 | 3.4 | 5.4 |
| X100 | 30 | 30 |

### Beispiel 5

In einem Glaskolben werden 63 g Testosteronundekanoat in 130 ml Aceton gelöst und auf 18°C gekühlt. Es wird ein Rotor-Stator Dispergierwerkzeug (Ultra Turrax, T25 basic, mit S25N-25F) eingebracht und mit einer Drehzahl von 12000-16000 U/min betrieben. Nach 1 min setzt Kristallisation ein. Der Ultra Turrax wird noch weitere 10 min betrieben und dann abgestellt. Die Primärkomsuspension wird anschließend mit einem Zyklus auf 21°C aufgeheizt und danach innerhalb von 30 min auf 5°C abgekühlt. Die Suspension wird filtriert und mit Hexan gewaschen.

Der Filterkuchen wird mit Luft trockengesaugt.

Es wurden Mikrokristalle mit folgender Partikelgrößenverteilung erhalten:

| | Partikelgröße(µm) | |
|---|---|---|
| | Primärkom (µm) | 1. Zyklus (µm) |
| X10 | 6 | 17 |
| X50 | 21 | 41 |
| X99 | 100 | 100 |
| X100 | 120 | 120 |

### Beispiel 6

In einem Glaskolben werden 13 g Gestoden in 130 ml Essigester/Ethanol (2.3 % Vol) - Gemisch gelöst und auf 35°C gekühlt. Es wird ein Rotor-Stator Dispergierwerkzeug (Ultra Turrax, T25 basic, mit S25N-25F) eingebracht und mit einer Drehzahl von 22000 U/min betrieben. Nach 1 min setzt Kristallisation ein. Der Ultra Turrax wird noch weitere 10 min betrieben und dann abgestellt. Die Primärkomsuspension wird anschließend auf 45°C aufgeheizt und danach innerhalb von 30 min auf 15°C abgekühlt. Die Suspension wird filtriert und mit Hexan gewaschen.

Der Filterkuchen wird mit Luft trockengesaugt.

Es wurden Mikrokristalle mit folgender Partikelgrößenverteilung erhalten:

| | Partikelgröße(µm) | |
|---|---|---|
| | Primärkorn (µm) | Ende (µm) |
| X10 | 4 | 8 |
| X50 | 15 | 21 |
| X99 | 51 | 51 |
| X100 | 61 | 61 |

### Beispiel 7

In einem Glaskolben werden 28 g Norethisteronacetat in 140 ml Methanol gelöst und auf 29°C gekühlt. Es wird ein Rotor-Stator Dispergierwerkzeug (Ultra Turrax, T25 basic, mit S25N-25F) eingebracht und mit einer Drehzahl von 22000 U/min betrieben. Nach 1 min setzt Kristallisation ein. Der Ultra Turrax wird noch weitere 10 min betrieben, die Suspension beprobt und dann abgestellt. Die Primärkornsuspension wird anschließend auf 34°C aufgeheizt und danach innerhalb von 1h, 15 min auf 5°C abgekühlt. Die Suspension wird filtriert und mit Hexan gewaschen.

Der Filterkuchen wird mit Luft trockengesaugt.

Es wurden Mikrokristalle mit folgender Partikelgrößenverteilung erhalten:

| | Partikelgröße(µm) | |
|---|---|---|
| | Primärkorn (µm) | Ende (µm) |
| X10 | 4 | 8,5 |
| X50 | 14 | 30,4 |
| X99 | 55 | 87 |
| X100 | 87 | 100 |

### Beispiel 8

In einem Glaskolben werden 50 g Methylnortestosteron werden in 250 g Ethanol gelöst und auf 20°C gekühlt. Es wird ein Rotor-Stator Dispergierwerkzeug (Ultra Turrax, T25 basic, mit S25N-25F) eingebracht und mit einer Drehzahl von 22000 U/min betrieben Gleichzeitig werden 375 ml Wasser zugegeben. Es setzt sofortige Kristallisation ein. Der Ultra Turrax wird noch weitere 10 min betrieben und dann abgestellt. Die Primärkornsuspension wird anschließend auf 21°C abgekühlt. Die Suspension wird filtriert und mit Wasser gewaschen, in Wasser zu einer 10%igen Suspension aufgeschlämmt und sprühgetrocknet

Es wurden Mikrokristalle mit folgender Partikelgrößenverteilung erhalten:

| | Partikelgröße(µm) | |
|---|---|---|
| | Kristallsusp.(µm) | Sprühtrocknung |
| X10 | 1.32 | 1.36 |
| X50 | 3.96 | 3.94 |
| X99 | 14 | 14 |
| X100 | 18 | 18 |

### Beispiel 9

Herstellung von Hartgelatinekapsein mit mikrokristallinem J-956:

| **Substanz** | **Menge** |
|---|---|
| Carbamat J-956, mikrokristallin | 1,000 mg |
| Mikrokristalline Cellulose | 102,480 mg |
| Magnesiumstearat | 0,520 mg |
| Hartgelatinekapsel, Größe 3 | 1 Stück |
| Kapselfüllmasse | 104,000 mg |

Das mikrokristalline J-956 wird in einem geeignetem Mischer (z.B. Containermischer) mit der mikrokristallinen Cellulose gemischt. Das Magnesiumstearat wird zugegeben und nochmals gemischt. Die Abwesenheit von Wasser in den Geräten ist zu prüfen.
Die Mischung wird mit einer geeigneten Kapselfüllmaschine (z.B. Harro Höflinger, KFMIIIC) in Hartgelatinekapsein Größe 3 abgefüllt.

### Beispiel 10

Herstellung von Weichgelatinekapseln mit Testosteronundekanoat

| | Kapselfüllgut: | | |
|---|---|---|---|
| 1. | Testosteronundekanoat, mikrokristallin | 40,000 | mg |
| 2. | Ölsäure | 210,000 | mg |
| | Kapselhülle: | | |
| 3. | Propylenglykol | 22,53 | mg |
| 4. | Glycerol 85 % | 28,66 | mg |
| 5. | Gelatine | 101,37 | mg |
| 6. | Titandioxid | 1,42 | mg |
| 7. | Eisenoxidgelb | 0,11 | mg |

Das Testosteronundekanoat ist in der Ölsäure zu lösen. Die Lösung wird in Weichkapseln Gr. 5 oval abgefüllt.

### Beispiel 11

Herstellung von Tabletten mit Norethisteronacetat

| | | | | |
|---|---|---|---|---|
| | | | | |
| 8. | Norethisteronacetat, mikrokristallin | Ph.Eur. | 1,000 | mg |
| 9. | Lactose-Monohydrat | Ph.Eur. | 82,000 | mg |
| 10. | Kartoffelstärke | Ph.Eur. + Zusatzforderung | 36,450 | mg |
| 11. | Gelatine | Ph.Eur. | 1,350 | mg |
| 12. | Talkum | Ph.Eur. | 5,400 | mg |
| 13. | Magnesiumstearat | Ph.Eur. | 0,700 | mg |
| 14. | Carboxymethylstärke-Natrium (Typ A) | Ph.Eur. | 2,700 | mg |
| 15. | Gereinigtes Wasser als 4,0 % Trocknungsverlust | | 5,400 | mg |

Das mikrokristalline Norethisteronacetat wird mit Lactose und Kartoffelstärke in einem Wirbelschichtgranulator mit einer wäßrigen Gelatinelösung granuliert. Das Granulat wird mit Magnesiumstearat und Talkum gemischt und auf Rundläuferpressen zu Tabletten einer Masse von 135 mg verpresst.

## Patentansprüche

1. Verfahren zum Herstellen von Kristallen von Steroiden, deren durchschnittliche Partikelgröße 1 µm bis 25 µm betragen und deren maximale Partikelgrößen 100 µm nicht überschreiten, wobei eine übersättigte Lösung des Steroids während der Kristallisation einem Naßmahlen mittels einer Vorrichtung zum Naßmahlen unterzogen wird, wodurch eine Primärkomsuspension erhalten wird.

2. Verfahren nach Anspruch 1, wobei die übersättigte Lösung 1 Gew.-% bis 50 Gew.-% des Steroids, bezogen auf die übersättigte Losung, in einem Lösungsmittel enthält.

3. Verfahren nach einem der vorhergehenden Ansprüche, wobei die übersättigte Lösung hergestellt wird durch Auflösen des Steroids in einem Lösungsmittel bei einer Temperatur unterhalb des Siedepunkts des Lösungsmittels und nachfolgendem Abkühlen auf eine Temperatur oberhalb des Gefrierpunkts der Lösung.

4. Verfahren nach einem der vorhergehenden Ansprüche, wobei die Kristallisation in einem einen Rührer aufweisenden Gefäß durchgeführt wird.

5. Verfahren nach einem der vorhergehenden Ansprüche, wobei die Vorrichtung zum Naßmahlen ein Rotor-Stator-Werkzeug, eine Rührwerksmühle, ein Walzenstuhl oder eine Kolloidmühle ist.

6. Verfahren nach einem der vorhergehenden Ansprüche, wobei die Primärkomsuspension auf eine Temperatur Tₘₐₓ unterhalb der Löslichkeitsgrenze der Primärkömer in der Suspension erwärmt und nachfolgend auf eine Temperatur Tₘᵢₙ oberhalb des Gefrierpunkts der Suspension abkühlt wird.

7. Verfahren nach Anspruch 6, wobei Tₘₐₓ so gewählt wird, das 10 Gew.-% bis 95 Gew.-% der Primärkömer im Lösungsmittel aufgelöst werden.

8. Verfahren nach einem der Ansprüche 6 oder 7, wobei Tₘᵢₙ so gewählt wird, daß die aufgelösten Primärkömer im wesentlichen wieder kristallisieren.

9. Verfahren nach einem der Ansprüche 6 bis 8, wobei das Abkühlen von Tₘₐₓ auf Tₘᵢₙ während 1 Minute bis 10 Std. erfolgt.

10. Verfahren nach einem der Ansprüche 6 bis 9, wobei das Erwärmen auf Tₘₐₓ und das Abkühlen auf Tₘᵢₙ 1 bis 20 mal durchgeführt wird.

11. Kristalle von Steroiden, erhältlich nach einem Verfahren gemäß einem der Ansprüche 1 bis 10.

12. Pharmazeutische Formulierung, die nach einem Verfahren gemäß einem der Ansprüche 1 bis 10 erhältliche Steroidkristalle aufweist.

## Claims

1. Method for making steroid crystals, said steroid crystals having a mean particle size in the range of 1 µm to 25 µm and a maximum particle size that does not exceed 100 µm, said method comprising subjecting a supersaturated solution of the steroid to wet milling by a wet milling apparatus while crystallising, in order to obtain a primary particle suspension.

2. Method according to claim 1, wherein said supersaturated solution contains in the range of 1 wt% to 50 wt% of said steroid in a solvent, based on said supersaturated solution.

3. Method according to one of the preceding claims, wherein said supersaturated solution is made by dissolving said steroid in a solvent at a temperature below the boiling point of said solvent and subsequent cooling to a temperature above the freezing point of the solution.

4. Method according to one of the preceding claims, wherein said crystallising is carried out in a vessel or container having a stirring device.

5. Method according to one of the preceding claims, wherein said wet milling apparatus is a rotor-stator apparatus, a stirring mill, a roller mill or a colloid mill.

6. Method according to one of the preceding claims, wherein the primary particle suspension is heated to a temperature Tₘₐₓ below the solubility limit of the primary particles in the suspension and is subsequently cooled to a temperature Tₘᵢₙ above the freezing point of the suspension.

7. Method according to claim 6, wherein Tₘₐₓ is selected so that 10 wt% to 95 wt% of said primary particles are dissolved in said solvent.

8. Method according to one of claims 6 or 7, wherein Tₘᵢₙ is selected so that the dissolved primary particles are substantially re-crystallised.

9. Method according to one of claims 6 to 8, wherein the cooling from Tₘₐₓ to Tₘᵢₙ occurs during a time interval in the range of 1 minute to 10 hours.

10. Method according to one of claims 6 to 9, wherein the heating to Tₘₐₓ and cooling to Tₘᵢₙ is carried out between 1 and 20 times.

11. Steroid crystals obtained by a method according to one of the claims 1 to 10.

12. Pharmaceutical formulation containing steroid crystals obtained by a method according to one of the claims 1 to 10.

## Revendications

1. Procédé de préparation de cristaux de stéroïdes, dont la taille de particules moyenne atteint de 1 µm à 25 µm et dont la taille de particules maximale ne dépasse pas 100 µm, où une solution sursaturée de stéroïde est soumise pendant la cristallisation à un broyage par voie humide au moyen d'un dispositif permettant le broyage par voie humide, moyennant quoi une suspension de grains primaires est obtenue.

2. Procédé selon la revendication précédente, où la solution sursaturée contient de 1 % en poids à 50 % en poids de stéroïde, par rapport à la solution sursaturée, dans un solvant.

3. Procédé selon l'une des revendications précédentes, où la solution sursaturée est préparée par dissolution du stéroïde dans un solvant à une température située en dessous du point d'ébullition du solvant et par refroidissement consécutif à une température située au-delà du point de congélation de la solution.

4. Procédé selon l'une des revendications précédentes, où la cristallisation s'effectue dans un récipient présentant un agitateur.

5. Procédé selon l'une des revendications précédentes, où le dispositif permettant le broyage par voie humide est un outil à rotor - stator, un broyeur agitateur, un moulin à cylindres ou un broyeur colloïdal.

6. Procédé selon l'une des revendications précédentes, où la suspension de grains primaires est chauffée à une température Tₘₐₓ située en dessous du seuil de solubilité des grains primaires en suspension et refroidie ensuite à une température Tₘᵢₙ qui se situe au-delà du point de congélation de la suspension.

7. Procédé selon la revendication 6, où Tₘₐₓ est ainsi choisie que 10 % en poids à 95 % en poids des grains primaires sont dissous dans le solvant.

8. Procédé selon l'une des revendications 6 ou 7, où Tₘᵢₙ est ainsi choisie que les grains primaires dissous se recristallisent sensiblement.

9. Procédé selon l'une des revendications 6 à 8, où le refroidissement de Tₘₐₓ à Tₘᵢₙ s'effectue en 1 minute à 10 heures.

10. Procédé selon l'une des revendications 6 à 9, où le chauffage à Tₘₐₓ et le refroidissement à Tₘᵢₙ sont effectués de 1 à 20 fois.

11. Cristaux de stéroïdes, pouvant être obtenus selon l'une des revendications 1 à 10.

12. Formulation pharmaceutique qui présente des cristaux de stéroïdes, pouvant être obtenus d'après un procédé selon l'une des revendications 1 à 10.
